# EUROPÄISCHE PATENTSCHRIFT

(11) **EP 1 896 089 B1**
(45) Veröffentlichungstag und Bekanntmachung des Hinweises auf die Patenterteilung: **19.12.2012**
(21) Anmeldenummer: 06761706.8
(22) Anmeldetag: 26.06.2006
(51) Int. Cl.: A61L 31/12, A61L 31/18

(54) **STABFÖRMIGER KÖRPER**
ROD-SHAPED BODY
CORPS EN FORME DE TIGE

(30) Priorität: 28.06.2005 DE 102005030472
(43) Veröffentlichungstag der Anmeldung: 12.03.2008
(73) Patentinhaber: MaRVis Technologies GmbH, 52068 Aachen (DE)
(72) Erfinder: PFEFFER, Joachim-Georg, 52070 Aachen (DE); GUNTHER, Rolf W., 52074 Aachen (DE); SCHMITZ RODE, Thomas, 52070 Aachen (DE)
(74) Vertreter: Ganahl, Bernhard
(86) Internationale Anmeldenummer: PCT/DE2006/001094
(87) Internationale Veröffentlichungsnummer: WO 2007/000148

(56) Entgegenhaltungen:
- EP-A- 1 206 945
- EP-A1- 1 388 346
- WO-A-03/000307
- DE-A1- 10 107 750
- US-A- 5 154 179
- US-A1- 2003 055 449
- M.K. KONINGS ET AL.: "Catheters and guidewires in interventional MRI: problems and solutions" MEDICA MUNDI, Bd. 45, Nr. 1, 1. März 2001 (2001-03-01), Seiten 31-39, XP002453987

## Beschreibung

Die Erfindung betrifft das Gebiet der bei minimal invasiven Eingriffen einzusetzenden Instrumente, insbesondere Führungsdrähte und Katheter.

Minimal invasive Eingriffe im menschlichen Körper erfordern Führungsdrähte und Katheter. Diese stehen in einer Vielzahl von Formen, Größen, Konfigurationen und mechanischen Eigenschaften für röntgendurchleuchtungsgesteuerte Verfahren zur Verfügung.

Für magnetresonanztomografisch (MRT) gesteuerte Prozeduren sind diese Katheter und Führungsdrähte allerdings nicht verwendbar, da sie in der Regel Metalle enthalten. Damit erzeugen sie Artefakte, die eine Bildbeurteilung erschweren oder unmöglich machen. Zudem bergen sie die Gefahr der induktiven Erhitzung im Magnetfeld und stellen somit eine mögliche Gefährdung für den Patienten dar.

Drei wesentliche Anforderungen an das Instrumentarium müssen erfüllt sein, damit eine Eignung in der MRT gegeben ist:
1. Es darf keine lang gestreckten Metallteile, wie z.B. Metallgewebe zur Katheterverstärkung oder Drahtseelen bei Führungsdrähten beinhalten.
2. Es sollte möglichst über der gesamten Länge in der MRT-Bildakquisition abbildbar sein, so dass die Position des Instruments in Beziehung zu dem bzw. den Organ(en) deutlich wird.
3. Da die örtliche Auflösung der Echtzeit-MRT derzeit eine direkte Darstellung von Kathetern und Führungsdrähten nicht ermöglicht, müssen im MRT-Bild gut sichtbare Effekte entlang des Instrumentariums erzeugt werden.

Solche Effekte sollten einerseits so groß sein, dass sich das Instrumentarium zwar gut im MRT-Bild abbildet, und anderseits so klein, dass wichtige Nachbarstrukturen dadurch nicht unkenntlich gemacht werden.

Das starke Magnetfeld eines Kernspintomographen macht einen fehlenden Ferromagnetismus zur Voraussetzung für den Einsatz von Instrumenten innerhalb dieses Gerätes. Dies schließt zum Beispiel viele Standardkatheter aus, die durch das starke Magnetfeld angezogen und umgelenkt werden können. Materialien, die alleine aus Kunststoffen gefertigt werden, erfüllen die Voraussetzung des fehlenden Ferromagnetismus.

Die Kleinheit interventionell eingesetzter Instrumente wie Führungsdrähte oder Katheter wirft im Rahmen der Magnetresonanztomographie und insbesondere im Rahmen von schneller Bildgebung ein besonderes Problem auf. Je schneller die Datenaufnahme im MRT erfolgt, desto geringer ist die örtliche Auflösung. Will man also einen so kleinen Gegenstand wie einen Katheter, der sich wegen der relativen Armut an Wasserstoffprotonen dunkel abbildet, in der MRT darstellen, sind entsprechen hochauflösende und somit auch langsame Bilder notwendig. Des Weiteren ist es sehr schwierig einen derartig kleinen Gegenstand bei den normalerweise angefertigten Schichtdicken von um 10 mm so abzubilden, dass er zum einen innerhalb der aufgenommenen Schicht liegt und zum anderen nicht durch Partialvolumeneffekte unsichtbar wird.

Ein Lösungsansatz hierfür liegt in Markierungen, die zu einer lokalen Signalauslöschung im MRT-Bild führen und somit das Instrument leichter auffindbar und darstellbar machen. Hierzu sind prinzipiell Materialien geeignet, die eine von Wasser unterschiedliche Suszeptibilität (Magnetisierbarkeit) aufweisen. Um Abhängigkeiten dieser Markierung von der Orientierung eines Instruments zum Magnethauptfeld zu vermeiden, müssen diese Markierungen lokal angebracht werden. Seltene Erden, die auch als MRT-Kontrastmittel genutzt werden, wurden in höherer Konzentration hierfür verwand. Sie wurden sowohl lokal als Markierungen als auch als Katheterfüllungen an- bzw. eingebracht, um eine bessere Sichtbarkeit von Instrumenten im MRT zu erreichen.

Ein wesentlicher Nachteil der bisher zur lokalen Markierung verwendeten Substanzen besteht in der relativ großen Masse, die notwendig ist, um einen im MRT ausreichenden Markierungseffekt zu erreichen. Dies spiegelt sich zum Beispiel in der Tatsache wider, dass wesentlich kompliziertere Techniken, die Strom durch einen Draht entlang dem Instrument leiten oder die Mikrospulen auf Katheter montieren, entwickelt wurden, obwohl hierdurch bedingte Sicherheitsprobleme wie Erhitzungen durch das Radiofrequenzfeld noch in keiner Weise gelöst sind. Derartige Erhitzungen treten auf, wenn elektrische Leiter, wie z.B. Metalle, über eine längere Strecke im MR-Tomographen den Radiofrequenzfeldern ausgesetzt werden. Kommt es zu einer Resonanz, so erfolgt über eine stehende Welle eine Summation der eingestrahlten Radioenergien mit dem möglichen Effekt einer wesentlichen Erhitzung des Leiters.

Bekannte einsträngige, homogene Nicht-Metall-Materialien weisen im Vergleich zur üblichen Metallseele wesentliche Nachteile in den Materialeigenschaften, hinsichtlich Stabilität, Biegsamkeit und Elastizität auf. So weisen z. B. Materialien mit hoher Stabilität zumeist eine geringe Biegsamkeit und/oder Elastizität auf. Deshalb ist es bisher auf einfachem Wege nicht gelungen, die gängigen Metallseelen durch einen MRT-kompatiblen und im MRT sichtbaren Werkstoff zu ersetzen.

In der US 2003/0055449 A1 ist ein MRI-sichtbarer Katheterballon beschrieben, der aus einem polymeren Material besteht, in dem ein ferromagnetisches Material verteilt ist. Das ferromagnetische Material besteht beispielsweise aus Eisenoxid. Das ferromagnetische oder paramagnetische Material besitzt typischerweise eine Teilchengröße von etwa 0,01 bis etwa 50 µm.

Die US 5,154,179 offenbart einen Katheter. Zur Sichtbarmachung des Katheters bei der Magnetresonanztomografie werden beim Extrudieren des schlauchförmigen Katheterkörpers geeignete ferromagnetische Partikel in den extrudierten Kunststoff eingebettet. Diese ferromagnetischen Partikeln erzeugen Artefakte bei der Magnetresonanztomografie.

In der DE 101 07 750 A1 ist ein kernspintauglicher Führungsdraht beschrieben, der eine metallische Seele aufweist, die z. B. aus Nickel-Titan ausgebildet ist. Um die Seele sind dünne Drähte verseilt. Diese Drähte bestehen aus einem nichtelektrisch leitfähigen Kunststoff, Glasfaser oder Kohlefaser. Über diese Struktur wird ein Kunststoff extrudiert. Aus der so gewonnenen Struktur wird die Seele entfernt und eine Seele aus einem metallischen Vorderteil und einem elektrisch nichtleitenden Teil hineingeschoben. Die Drähte bewirken die Festigkeit in Richtung Druck und Zug sowie bzgl. der Drehung.

Aus der EP 1 388 346 A1 geht ein Ballonkatheter hervor, der mit kurzen Fasern verstärkt ist. Hierbei ist der Ballon aus einem Verbundmaterial ausgebildet, das kurze Fasern zum Verstärken einer Kunststoffmatrix enthält: Aufgabe Der hier beschriebenen Erfindung liegt daher die Aufgabe zugrunde, MRT-kompatible Instrumente, insbesondere einen Katheter und einen Führungsdraht zu realisieren, der keine längeren zusammenhängenden Metallteile aufweist und somit nicht der Gefahr einer induktiven Erhitzung unterliegt, andererseits jedoch über der ganzen Länge längs und quer zur Hauptmagnetisierung in geeigneten MRT-Sequenzen darstellbar ist, ohne dass die Körperstrukturen in der Umgebung des Instruments durch Artefakte unkenntlich gemacht werden. Es ist weiter Aufgabe der vorliegenden Erfindung, MRT-kompatible Instrumente anzugeben, die gegenüber den bislang bekannten Instrumenten keinerlei Einschränkung hinsichtlich ihrer Materialeigenschaften und der damit verbundenen Handhabbarkeit aufweisen.

### Lösung

Zur Lösung dieser Aufgabe wird zunächst ein stabförmiger Körper ("Stab"). vorgeschlagen, der aus einem oder mehreren nicht-metallischen Filamenten und einem nicht-ferromagnetischen Matrixwerkstoff besteht, wobei der Matrixwerkstoff das oder die Filamente umschließt und/oder miteinander verklebt, und wobei in den Matrixwerkstoff eine Dotierung aus magnetresonanztomografisch Artefakte erzeugenden Partikeln eingebracht ist. Die Kombination aus dem Matrixwerkstoff mit dem oder den nicht-metallischen Filament(en) erzielt auf überraschend einfache Weise metallähnliche Steifigkeits-, Biegsamkeits- und Elastizitätseigenschaften.

Die den stabförmigen Körper bildenden Filamente sind leicht und kostengünstig herstellbar, dabei ausreichend stabil und können Druck- und Zugkräfte sowie Drehmomente übertragen. Durch die Dotierung des zum Umschließen eines einzelnen Filamentes oder zum Verkleben mehrerer Filamente ohnehin erforderlichen Matrixwerkstoffes ist eine einfache und effektive Möglichkeit geschaffen, den stabförmigen Körper in der MRT darzustellen und gleichzeitig eine dem heute üblichen Standard entsprechende Handhabbarkeit zu gewährleisten.

Aus diesem Grundelement werden, wie weiter unten beschrieben ist, die eigentlichen Instrumente wie Katheter, Führungsdrähte etc. aufgebaut.

Vorteilhafte Ausgestaltungen der Erfindung sind Gegenstand der Unteransprüche.

Besonders vorteilhaft ist es, wenn die Filamente aus Kunststoff und/oder Glasfaser bestehen. Derartige Filamente können leicht und preiswert, in großen Längen, mit unterschiedlichsten Querschnitten und Dicken hergestellt werden. Insbesondere Glasfaser verfügt über geringste Dehnungen, weshalb eine sehr direkte Kraft- und Momentenübertragung möglich ist.

Der Matrixwerkstoff kann vorteilhafter Weise aus Epoxidharz bestehen. Epoxidharze stehen in einer großen Fülle von Eigenschaften zur Verfügung und die zur Verarbeitung erforderlichen Geräte sind ausgereift.

Die Stäbe können entlang ihrer Längsachse kontinuierlich mit magnetresonanztomografisch Artefakte erzeugenden Partikeln dotiert sein. Hierdurch wird der Stab auf seiner gesamten Länge in der MRT gut sichtbar.

Für manche Anwendungen kann es jedoch auch bevorzugt sein, den Stab entlang seiner Längsachse diskontinuierlich, insbesondere abschnittsweise mit magnetresonanztomografisch Artefakte erzeugenden Partikeln zu dotieren. Dies trifft insbesondere für die Spitzen der Stäbe zu, die in manchen Fällen besonders gut sichtbar sein sollen.

Innerhalb der Stäbe können die Filamente parallel verlaufend angeordnet sein. Hierdurch wird eine besonders einfache Verarbeitung unterstützt.

Die Filamente können nach besonderes bevorzugten Ausführungsformen jedoch auch miteinander verflochten, verwoben, vernetzt, verdrillt oder wendelförmig verlaufend angeordnet sein, um bestimmte gewünschte Eigenschaften, insbesondere mechanische Eigenschaften zu erzielen.

Vorteilhaft ist es, wenn die Masse eines einzelnen Partikels im Mikro- bis Nanogramm-Bereich liegt und aufgrund der im Vergleich zum Matrixwerkstoff geringen Menge die äußere Form, Stabilität und die Torquierungseigenschaften des Stabs im Wesentlichen nicht beeinflusst.

Typische bevorzugte Größen der Stäbe liegen im Bereich eines Durchmessers zwischen 0,005 mm und 5 mm, vorzugsweise zwischen 0,1 und 1 mm.

Aus den beschriebenen Stäben wird ein zylindrischer Verbindungskörper ("Zylinder"), insbesondere ein Führungsdraht, gebildet, und zwar dergestalt, das mindestens ein Stab von einem nicht-ferromagnetischen Matrixwerkstoff umschlossen wird bzw. mehrere Stäbe mit einem nicht-ferromagnetischen Matrixwerkstoff verklebt und/oder umschlossen werden.

Auf diese Weise können mit ein und dem selben Element (dem Stab) und dem Matrixwerkstoff sehr leicht und kostengünstig Zylinder unterschiedlichster Geometrien und mechanischer sowie MRT-Eigenschaften aufgebaut werden.

Der Zylinder kann z. B. besonderes einfach aus Stäben gleichen Durchmessers bestehen oder - nach einer anderen Ausführungsform - aus Stäben unterschiedlichen Durchmessers. Im letzteren Falle können insbesondere um einen ersten Stab herum zweite Stäbe geringeren Durchmessers angeordnet sein.

Die einzelnen Stäbe können - wie zuvor die Filamente - miteinander verflochten, verwoben, vernetzt, verdrillt oder wendelförmig verlaufend angeordnet sein.

Besonders bevorzugt ist es, wenn die Zylinder Stäbe unterschiedlicher magnetresonanztomografischer Eigenschaften aufweisen. So kann ein und der selbe Zylinder (z. B. als Führungsdraht) in unterschiedlichen MRT-Sequenzen (zum Beispiel zur spezifischen Darstellung von Fettgewebe, Muskelgewebe usw.) gleich gut beobachtet werden.

Vorteilhaft ist es, die äußere Oberfläche des Zylinders hydrophil zu beschichten und ihn damit körperverträglich zu machen.

Aus den beschriebenen Stäben können jedoch auch andere Instrumente gefertigt werden, insbesondere ein schlauchförmiger Verbindungskörper ("Katheter"). Dieser besteht aus mindestens einem Stab, der von einem nicht-ferromagnetischen Matrixwerkstoff umschlossen wird und/oder mehrerer Stäbe miteinander verklebt und/oder umschließt.

Nach einer Ausführungsform des Katheters besteht dieser aus mehreren, in seiner Wandung auf dem Umfang radiär verteilt eingebetteten Stäben. Diese können insbesondere zur Erzielung symmetrischer Eigenschaften regelmäßig radiär verteilt eingebettet sein.

Aus dem gleichen Grunde können die den Katheter bildenden Stäbe gleichen Durchmesser aufweisen, in besonderen Fällen können aber auch Stäbe unterschiedlichen Durchmessers zur Anwendung kommen.

Ebenso wie bei den Zylindern / Führungsdrähten können auch bei dem Katheter die Stäbe miteinander verflochten, verwoben, vernetzt, verdrillt oder wendelförmig verlaufend angeordnet sein, die Stäbe unterschiedliche magnetresonanztomografische Eigenschaften aufweisen und seine äußere Oberfläche hydrophil beschichtet sein.

Neben den hier beschriebenen Instrumenten können aus den stabförmigen Körpern und dem Matrixwerkstoff in gleicher Weise auch andere Instrumente erstellt werden, z. B. Dormia-Fangkörbchen.

Nachstehend werden bevorzugte Ausführungsformen der Erfindung anhand der beigefügten schematischen Zeichnungsfiguren erläutert, die folgendes zeigen:
Fig. 1 einen dotierten Stab,
Fig. 2 einen zylindrischen Verbindungskörper (Führungsdraht),
Fig. 3 einen schlauchförmigen Verbindungskörper (Katheter),
Fig. 4 einen Querschnitt durch einen dotierten Stab, und
Fig. 5 die Spitze eines Führungsdrahtes.

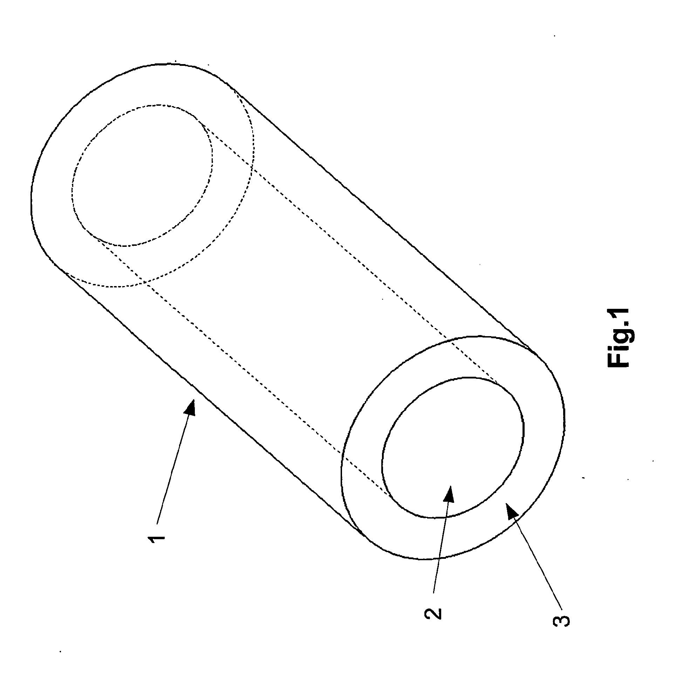

Der in Figur 1 gezeigte Stab 1 bzw. Stabausschnitt besteht aus einem langgestreckten Glasfaserfilament 2, welches in Epoxidharz 3 als Matrixwerkstoff eingebettet ist. Der Stab 1 kann durch herkömmliche Verfahren, insbesondere durch Extrusion praktisch endlos hergestellt werden. Nach der Extrusion wird er gegebenenfalls auf für die Weiterverarbeitung geeignete Längen geschnitten.

In das Epoxidharz eingebracht sind - nicht dargestellte - magnetresonanztomografisch Artefakte erzeugende Partikel, z. B. Nanopartikel. Diese sind in dem Matrixwerkstoff homogen verteilt, so dass sich ein entlang der Längsachse homogen dotierter Stab 1 ergibt.

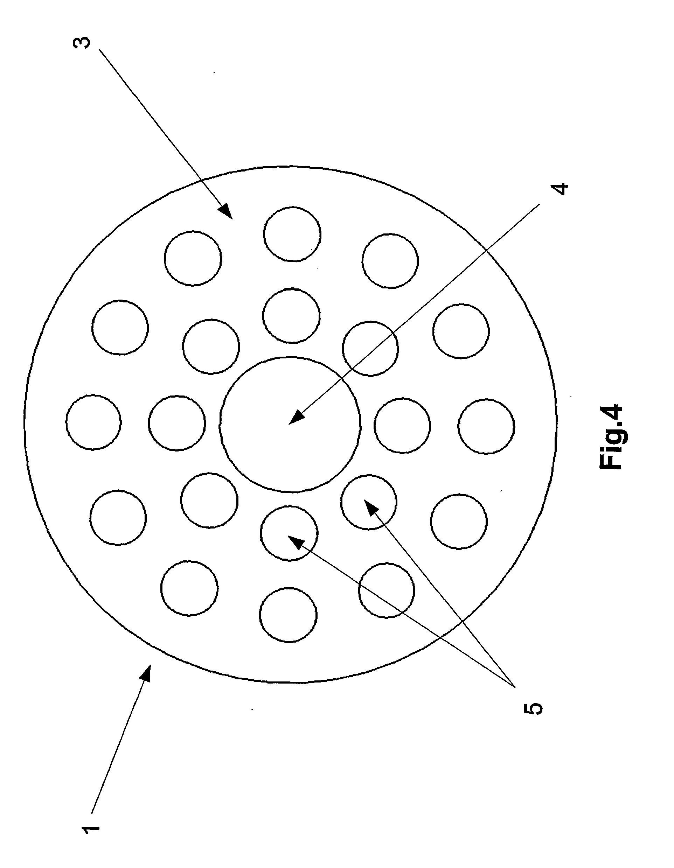

Wie Figur 4 zeigt, können anstelle eines Filamentes auch mehrere Filamente 4, 5 in einem stabförmigen Körper 1 angeordnet sein. In gezeigten Beispiel sind dies ein relativ dickes Filament 4 und darum angeordnete relativ dünne Filamente 5. Sämtliche Filamente 4, 5 werden mit dem Epoxidharz 3 verklebt und von diesem umschlossen.

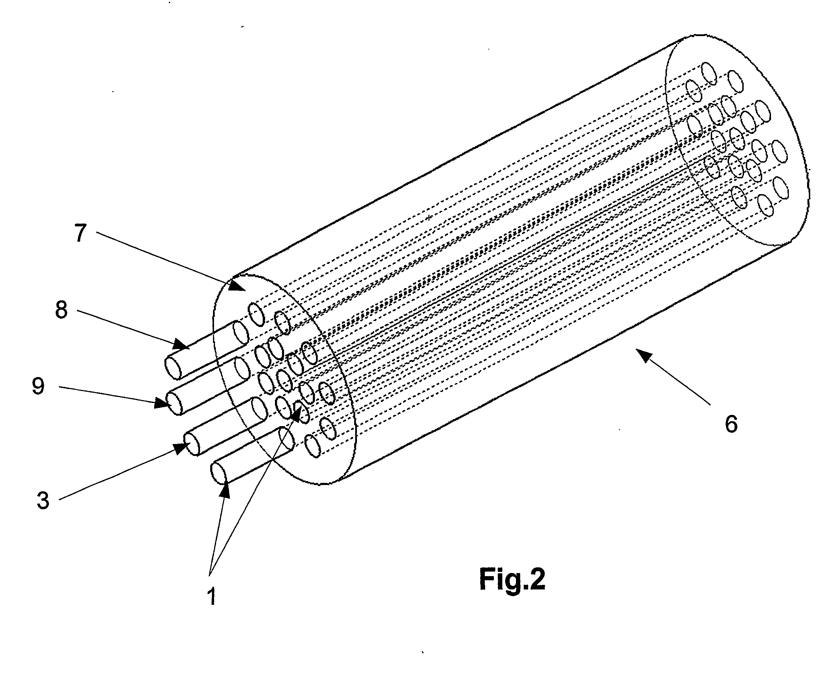

In Figur 2 ist ein zylindrischer Verbindungskörper 6 bzw. ein Abschnitt desselben gezeigt. Die Erstreckung in Längsrichtung kann erheblich länger sein und beträgt z. B. mehrere Meter bei einem Durchmesser von z. B. 0,1 mm.

Der zylindrische Verbindungskörper 6 ist aufgebaut aus mehreren Stäben 1, die von einem Matrixwerkstoff 7, z. B. Epoxidharz, verklebt und umschlossen werden. Dieser Matrixwerkstoff 7 ist, entgegen dem Matrixwerkstoff 3, nicht mit magnetresonanztomografisch Artefakte erzeugenden Partikeln dotiert. Die Sichtbarkeit des zylindrischen Verbindungskörpers 6 in der MRT beruht einzig auf der Sichtbarkeit der eingebetteten Stäbe 1. In der dargestellten Ausführungsform sind Stäbe 1, 8, 9 unterschiedlicher Dotierung eingearbeitet, so dass je nach Sequenz andere Stäbe 1, 8, 9 in der MRT sichtbar werden.

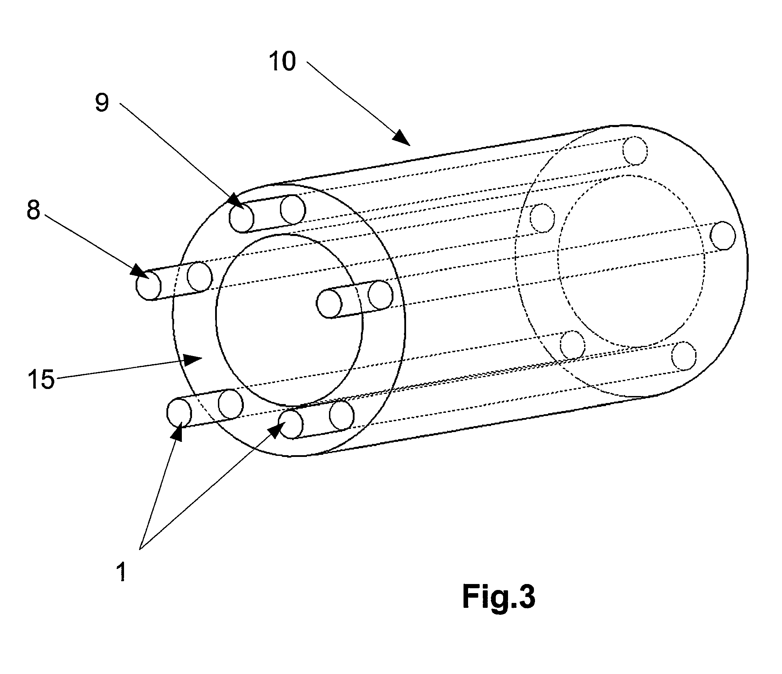

Auf die gleiche Weise kann, wie Figur 3 zeigt, auch ein schlauchförmiger Verbindungskörper 10 (dargestellt ist wiederum ein Abschnitt desselben) gebildet sein.

Der schlauchförmige Verbindungskörper 10 ist ebenfalls aufgebaut aus einem Mantelwerkstoff 15 und mehreren Stäben 1, 8, 9 unterschiedlicher Dotierung. Sämtliche Stäbe sind am Umfang des schlauchförmigen Verbindungskörpers 10 regelmäßig verteilt.

Sowohl der zylindrische Verbindungskörper 6 als auch der schlauchförmige Verbindungskörper 10 kann mit einer nicht dargestellten hydrophilen Beschichtung versehen sein.

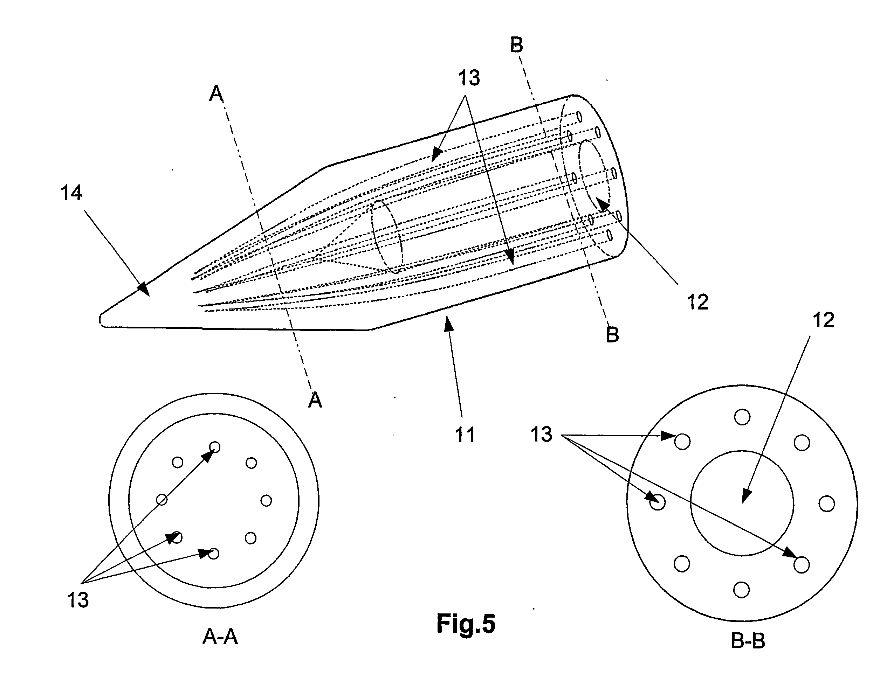

Die Enden der zylindrischen und schlauchförmigen Verbindungskörper 6, 10 können in geeigneter Weise bearbeitet sein, z. B. gerundet, poliert oder mit einem Endstück versehen. Wie Figur 5 zeigt, kann insbesondere bei einem Führungsdraht 11 mit einem inneren Stab 12 und darum radial verteilt angeordneten äußere Stäben 13 der innere Stab 12 früher enden als die äußeren Stäbe 13. Diese werden zusammengeführt und bilden eine Spitze 14 (Anordnung der Stäbe 12 und 13 wie in den Querschnitten A-A beziehungsweise B-B dargestellt).

## Patentansprüche

1. Stabförmiger Körper ("Stab") zur Fertigung eines MRT-kompatiblen medizinischen Instrumentes, bestehend aus
- einem oder mehreren nicht-metallischen Filamenten und
- einem nicht-ferromagnetischen Matrixwerkstoff
- wobei der Matrixwerkstoff das oder die Filamente umschließt und/oder miteinander verklebt, und
- einer in den Matrixwerkstoff eingebrachten Dotierung aus magnetresonanztomografisch Artefakte erzeugenden Partikeln.

2. Stab nach Anspruch 1, **dadurch gekennzeichnet, dass** die Filamente aus Kunststoff und/oder Glasfaser bestehen.

3. Stab nach Anspruch 1 oder 2, **dadurch gekennzeichnet, dass** der Matrixwerkstoff aus Epoxidharz besteht.

4. Stab nach einem oder mehreren der Ansprüche 1 bis 3, **dadurch gekennzeichnet, dass** er entlang seiner Längsachse kontinuierlich mit magnetresonanztomografisch Artefakte erzeugenden Partikeln dotiert ist.

5. Stab nach einem oder mehreren der Ansprüche 1 bis 3, **dadurch gekennzeichnet, dass** er entlang seiner Längsachse diskontinuierlich, insbesondere abschnittsweise mit magnetresonanztomografisch Artefakte erzeugenden Partikeln dotiert ist.

6. Stab nach einem oder mehreren der Ansprüche 1 bis 5, **dadurch gekennzeichnet, dass** die Filamente parallel verlaufend angeordnet sind.

7. Stab nach einem oder mehreren der Ansprüche 1 bis 5, **dadurch gekennzeichnet, dass** die Filamente miteinander verflochten, verwoben, vernetzt, verdrillt oder wendelförmig verlaufend angeordnet sind.

8. Stab nach einem oder mehreren der Ansprüche 1 bis 7, **dadurch gekennzeichnet, dass** die Masse eines einzelnen Partikels im Mikro- bis Nanogramm-Bereich liegt und aufgrund der im Vergleich zum Matrixwerkstoff geringen Menge die äußere Form, Stabilität und die Torquierungseigenschaften des Stabs im Wesentlichen nicht beeinflusst.

9. Stab nach einem oder mehreren der Ansprüche 1 bis 8, **dadurch gekennzeichnet, dass** sein Durchmesser im Bereich zwischen 0,005 mm und 5 mm, vorzugsweise zwischen 0,1 und 1 mm liegt.

10. Zylindrischer Verbindungskörper ("Zylinder"), bestehend aus
- mindestens einem Stab nach den Ansprüchen 1 bis 9, und
- einem nicht-ferromagnetischen Matrixwerkstoff,
- wobei der Matrixwerkstoff den oder die Stäbe umschließt und/oder miteinander verklebt.

11. Zylinder nach Anspruch 10, bestehend aus Stäben gleichen Durchmessers.

12. Zylinder nach Anspruch 10, bestehend aus Stäben unterschiedlichen Durchmessers.

13. Zylinder nach Anspruch 12, bestehend aus einem ersten Stab, um welchen herum zweite Stäbe geringeren Durchmessers angeordnet sind.

14. Zylinder nach den Ansprüchen 10 bis 13, **dadurch gekennzeichnet, dass** die Stäbe miteinander verflochten, verwoben, vernetzt, verdrillt oder wendelförmig verlaufend angeordnet sind.

15. Zylinder nach den Ansprüchen 10 bis 14, **dadurch gekennzeichnet, dass** die Stäbe unterschiedliche magnetresonanztomografische Eigenschaften aufweisen.

16. Zylinder nach den Ansprüchen 10 bis 15, **dadurch gekennzeichnet, dass** seine äußere Oberfläche hydrophil beschichtet ist.

17. Schlauchförmiger Verbindungskörper ("Katheter"), bestehend aus
- mindestens einem Stab nach den Ansprüchen 1 bis 9, und
- einem nicht-ferromagnetischen Matrixwerkstoff,
- wobei der Matrixwerkstoff den oder die Stäbe umschließt und/oder miteinander verklebt.

18. Katheter nach Anspruch 17, bestehend aus mehreren, in seiner Wandung auf dem Umfang radiär verteilt eingebetteten Stäben.

19. Katheter nach Anspruch 18, **dadurch gekennzeichnet, dass** die Stäbe regelmäßig radiär verteilt eingebettet sind.

20. Katheter nach den Ansprüchen 17 bis 19, bestehend aus Stäben gleichen Durchmessers.

21. Katheter nach den Ansprüchen 17 bis 19, bestehend aus Stäben unterschiedlichen Durchmessers.

22. Katheter nach den Ansprüchen 17 bis 21, **dadurch gekennzeichnet, dass** die Stäbe miteinander verflochten, verwoben, vernetzt, verdrillt oder wendelförmig verlaufend angeordnet sind.

23. Katheter nach den Ansprüchen 17 bis 22, **dadurch gekennzeichnet, dass** die Stäbe unterschiedliche magnetresonanztomografische Eigenschaften aufweisen.

24. Katheter nach den Ansprüchen 17 bis 23, **dadurch gekennzeichnet, dass** seine äußere Oberfläche hydrophil beschichtet ist.

## Claims

1. A rod-shaped body ("rod") for manufacturing an MRT-compatible medical instrument, consisting of
- one or more non-metallic filaments and
- a non-ferromagnetic matrix material
- the matrix material enclosing the one or more filaments and/or gluing them to each other, and
- a doping which is introduced into the matrix material and is made of particles generating artifacts under magnetic resonance imaging.

2. The rod according to claim 1, **characterized in that** the filaments consist of synthetic material and/or glass fiber.

3. The rod according to claim 1 or 2, **characterized in that** the matrix material consists of epoxy resin.

4. The rod according to one or more of claims 1 to 3, **characterized in that** it is continuously doped along its longitudinal axis with particles generating artifacts under magnetic resonance imaging.

5. The rod according to one or more of claims 1 to 3, **characterized in that** it is discontinuously doped along its longitudinal axis, in particular in sections, with particles generating artifacts under magnetic resonance imaging.

6. The rod according to one or more of claims 1 to 5, **characterized in that** the filaments are arranged so as to extend parallel to each other.

7. The rod according to one or more of claims 1 to 5, **characterized in that** the filaments are braided, interwoven, cross-linked, twisted or arranged so as to extend in spiral shape.

8. The rod according to one or more of claims 1 to 7, **characterized in that** the mass of an individual particle is in the microgram to nanogram range and essentially does not affect the outer shape, stability and the torsion characteristics of the rod due to the small amount compared to the matrix material.

9. The rod according to one or more of claims 1 to 8, **characterized in that** its diameter is in the range between 0.005 mm and 5 mm, in particular between 0.1 and 1 mm.

10. A cylindrical connection body ("cylinder"), consisting of
- at least one rod according to claims 1 to 9, and
- a non-ferromagnetic matrix material,
- the matrix material enclosing the one or more rods and/or gluing them to each other.

11. The cylinder according to claim 10, consisting of rods of equal diameter.

12. The cylinder according to claim 10, consisting of rods with different diameters.

13. The cylinder according to claim 12, consisting of a first rod which has second rods of smaller diameter arranged around it.

14. The cylinder according to claims 10 to 13, **characterized in that** the rods are braided, interwoven, cross-linked, twisted or arranged so as to extend in spiral shape.

15. The cylinder according to claims 10 to 14, **characterized in that** the rods have differing characteristics in terms of magnetic resonance tomography.

16. The cylinder according to claim 10, **characterized in that** its outer surface is provided with a hydrophilic coating.

17. A hose-shaped connection body ("catheter"), consisting of
- at least one rod according to claims 1 to 9, and
- a non-ferromagnetic matrix material,
- the matrix material enclosing the one or more rods and/or gluing them to each other.

18. The catheter according to claim 17, consisting of a plurality of rods which are embedded in its wall so as to be radially distributed on the circumference.

19. The catheter according to claim 18, **characterized in that** the rods are embedded so as to be regularly radially distributed.

20. The catheter according to claims 17 to 19, consisting of rods of equal diameter.

21. The catheter according to claims 17 to 19, consisting of rods with different diameters.

22. The catheter according to claims 17 to 21, **characterized in that** the rods are braided, interwoven, cross-linked, twisted or arranged so as to extend in spiral shape.

23. The catheter according to claims 17 to 22, **characterized in that** the rods have differing characteristics in terms of magnetic resonance tomography.

24. The catheter according to claims 17 to 23, **characterized in that** its outer surface is provided with a hydrophilic coating.

## Revendications

1. Corps en forme de tige ("tige") pour réaliser un instrument médical compatible avec MRT (= tomographie à résonance magnétique), composé de
- un ou plusieurs filaments non métalliques, et
- un matériau de matrice non ferromagnétique,
- le matériau de matrice entourant et/ou collant le ou les filaments l'un à l'autre, et
- un dopage de particules générant des artifices, par tomographie à résonance magnétique, introduites dans le matériau de matrice.

2. Tige selon la revendication 1, **caractérisée par le fait que** les filaments sont réalisés en matière plastique et/ou en fibre de verre.

3. Tige selon la revendication 1 ou 2, **caractérisée par le fait que** le matériau de matrice est réalisé en résine époxyde.

4. Tige selon l'une ou plusieurs des revendications 1 à 3, **caractérisée par le fait qu'**elle est dopée le long de son axe longitudinal en continu de particules générant des artifices par tomographie à résonance magnétique.

5. Tige selon l'une ou plusieurs des revendications 1 à 3, **caractérisée par le fait qu'**elle est dopée le long de son axe longitudinal de manière discontinue, en particulier par segments, de particules générant des artifices par tomographie à résonance magnétique.

6. Tige selon l'une ou plusieurs des revendications 1 à 5, **caractérisée par le fait que** les filaments sont disposés en s'étendant parallèles entre eux.

7. Tige selon l'une ou plusieurs des revendications 1 à 5, **caractérisée par le fait que** les filaments sont disposés en s'étendant tressés ou tissés, réticulés, torsionnés ou en méandre entre eux.

8. Tige selon l'une ou plusieurs des revendications 1 à 5, **caractérisée par le fait que** la masse d'une particule individuelle se situe dans la plage micrométrique ou nanométrique et, du fait de la faible quantité par rapport au matériau de matrice, n'a substantiellement pas d'influence sur la forme extérieure, la stabilité et les propriétés de torquage de la tige.

9. Tige selon l'une ou plusieurs des revendications 1 à 8, **caractérisée par le fait que** son diamètre est compris entre 0,005 et 5 mm, de préférence entre 0,1 et 1 mm.

10. Corps cylindrique de connexion ("cylindre"), composé de
- au moins une tige selon les revendications 1 à 9, et
- un matériau de matrice non ferromagnétique,
- le matériau de matrice entourant et/ou collant le ou les filaments l'un à l'autre.

11. Cylindre selon la revendication 10, composé de tiges de même diamètre.

12. Cylindre selon la revendication 10, composé de tiges de diamètre différent.

13. Cylindre selon la revendication 12, composé d'une première tige autour de laquelle sont disposées deux tiges de faible diamètre.

14. Cylindre selon les revendications 10 à 13, **caractérisé par le fait que** les tiges sont disposées en s'étendant tressées ou tissées, réticulées, torsionnées ou en méandre entre eux.

15. Cylindre selon les revendications 10 à 14, **caractérisé par le fait que** les tiges présentent des propriétés de tomographie à résonance magnétique différentes.

16. Cylindre selon les revendications 10 à 14, **caractérisé par le fait que** sa surface extérieure est à revêtement hydrophile.

17. Corps de connexion en forme de flexible ("cathéter"), composé de
- au moins une tige selon les revendications 1 à 9, et
- un matériau de matrice non ferromagnétique,
- le matériau de matrice entourant et/ou collant le ou les filaments l'un à l'autre.

18. Cathéter selon la revendication 17, composé de plusieurs tiges incorporées réparties radiairement en périphérie dans sa paroi.

19. Cathéter selon la revendication 18, **caractérisé par le fait que** les tiges sont incorporées réparties radiairement de manière régulière.

20. Cathéter selon les revendications 17 à 19, composé de tiges de même diamètre.

21. Cathéter selon les revendications 17 à 19, composé de tiges de diamètre différent.

22. Cathéter selon les revendications 17 à 21, **caractérisé par le fait que** les tiges sont disposées en s'étendant tressées ou tissées, réticulées, torsionnées ou en méandre entre eux.

23. Cathéter selon les revendications 17 à 22, **caractérisé par le fait que** les tiges présentent des propriétés de tomographie à résonance magnétique différentes.

24. Cathéter selon les revendications 17 à 23, **caractérisé par le fait que** sa surface extérieure est à revêtement hydrophile.
